# EUROPEAN PATENT APPLICATION

(11) **EP 1 168 771 A2**
(43) Date of publication of application: **02.01.2002**
(21) Application number: 01810501.5
(22) Date of filing: 18.05.2001
(51) Int. Cl.: H04L 29/06

(54) **System and method for transmitting information between doctors and hospitals**

(30) Priority: 19.05.2000 US 205872 P
(71) Applicant: SanaLink AG, 8702 Zollikon (CH)
(72) Inventor: Dubler, Andreas M., 8702 Zollikon (CH); Scherrer, Felix E., 8126 Zumikon (CH); Perren, Heinz G., 8053 Zürich (CH)
(74) Representative: Frei, Alexandra Sarah

(57) **Abstract**

A method of exchanging information between one of a plurality of doctors and one of a plurality of hospitals is disclosed. According to this method, on an operating platform, a data set containing hospital related information including information about hospital occupancy is maintained. On a client system of said one doctor, information relating to at least some of the plurality of hospitals including information from said data set is displayed. The doctor may then send a request signal to the operating platform, thereby generating a reservation order created by said operating platform. This reservation order is then sent to one of said plurality of hospitals, the hospital being specified by information contained in the request signal. Finally, depending on the successful transmittal of said reservation order, said data set containing hospital occupancy information is modified.

## Description

### FIELD OF THE INVENTION

The present invention relates to methods and systems for transmitting, securing and filtering information. It more specifically relates to a booking engine linking doctors, hospitals (or other medical related facilities) and insurance companies.

### BACKGROUND OF THE INVENTION

Currently, if a doctor wishes to reserve a bed, operating room or medical equipment for a patient, the doctor's secretary will phone a hospital administration department to inquire about availability. In particular, for emergency patients, this can be a nightmare because it may require the doctor's secretary to phone multiple hospital administration departments. The hospital administration departments, moreover, must deal with numerous phone calls regarding the patients of many different doctors. Thus, the current system in practice throughout the world creates an undue administrative burden on both doctors and hospitals.

Of course, also modern communication channels such as e-mails are used. However, on a technical level, all currently used communication channels work on a point-to-point basis: A connection between two participants is established, information exchange takes place unidirectionally or bi-directionally and then the connection is closed. Next to the above mentioned burden placed on the participants - communication between n hospitals and m doctors, e.g., requires n*m point-to-point communication channels - depending on the medium, also security problems arise.

Existing systems for exchanging information between internet users and hotels, taxis, warehouses etc. can not readily be adapted to be used for information exchange between doctors, hospital and insurance companies etc. since the security standards required for use in the medical field can not be met. In general and compared to existing information exchange systems, security is an important issue on at least four levels:
- Given the delicacy of some transmitted information, it has to be made very sure that no un-authorized third party gains access to information.
- Also, it has to be made sure that the information can only be accessed by the participant of the system to whom the information concerns an not by any other participant. This may be a complex requirement, since one compilation of data may contain information for different parties. The consequences of mistakes in the information transmittal or of wrongly placed information can be dire.
- Also, the addressee has to be certain that the data he receives stems from the right source and has not been manipulated.
- Finally, it may be crucial that there is certainty that a piece of information actually arrives at its destination. In the medical field, a failure in a reservation may be more than a nuisance and cause substantial discomfort for a patient. In case of emergencies, the security of data transmission may even be decisive between life and death.

For these reasons it would be desirable to have a system and a method for transmitting information as well as corresponding computer software which allow the information transmittal between hospital, doctors and insurance companies to have access to information relevant for them in a secure and economical way.

### SUMMARY OF THE INVENTION

It is therefore an object of the invention to provide a technical solution to the above described problems, and to provide a method and a system wherein data transfer between doctors and hospitals is ensured in a secure and economical manner. Preferably, insurance companies should be enabled to gain real time access to bookings relating to their patients so that approvals can be issued (or denied) in a faster and more cost-effective manner.

Specifically, the present invention provides a medical booking engine linking doctors/ hospitals (or other medical related facilities) and insurance companies which replaces the current complicated process of placing patients with a superior, modern communication platform.

According to the invention, therefore, an operating platform is provided comprising means for multiplexing information including means for encrypting data destination specifically.

The system receives from hospitals availability of different categories of beds, operating rooms, available services from the hospital and/or medical equipment. The system then enables authorized doctors to check availability in different hospitals by name, region, and/or radius. In addition, the doctor preferably provides the gender of the patient, the insurance class, the proposed length of stay, the diagnosis/ and the department of the hospital.

If there is availability for the requested bed, operating room, hospital service and/or medical equipment, the system enables the doctor to directly book the patient (whose name has not yet necessarily been given). Upon sending the booking, the system creates a reservation number for identification of the file, and adjusts the availability in the selected hospital.

The system also enables a doctor to provide a separate set of information relating to the requirements the doctor has for the hospital, such as, for example, relating to specific tests which are to be performed. Such information is provided to the hospital, which then must check and confirm that the specially requested items will be available.

Preferably, the system of the present invention also sends to the patient's insurance company a message with appropriate information relating to the case which will enable the insurance company to approve a foreseeable total cost amount with respect to each of the doctor and the hospital, and/or the patient. The insurance company may issue payment precertification on the spot and confirm that the patient has sufficient health care insurance coverage. In addition, if the insurance company wishes to obtain a second opinion, the system can transmit the case information to a third party doctor of confidence who can then communicate with the original doctor to get access to the patient file in further detail. In this manner, the insurance company can be provided with a better recommendation for approval or denial of the requested services and their costs.

It should be appreciated that the system can be applied to any medical related facility or service, including rehabilitation units, nursing homes, physiotherapy hours, etc.

It should also be appreciated that the communication platform over which the system may be set up can be the Internet or any other suitable communication network, and that the system may be accessed via a computer or any other suitable processing device such as a WAP enabled mobile phone. The system may also be set up to use more than one communication platform and incorporate the appropriate interfaces thereto.

The system comprises the operating platform with means for multiplexing information. Information can be distributed to different channels according to its importance, its security relevance, or according to its destination. The operating platform e.g. comprises means for encrypting information in a destination specific manner.

The multiplexing means as well as the encrypting means may be implemented by computer software. As an alternative, they may also be implemented by conventional (analog) electronics or by other means.

The compilation of data to be transmitted usually requires several steps. As an example, in a first step a doctor requests information on a hospitals he considers placing his patient in. Then he chooses the hospital. In a next step he compiles the information about the patient as well as data such as the estimated time of his stay and possibly some information about the diseases/injuries of the patient. Finally, he places a order to be transmitted to the hospital accompanied by a possible notification to the insurance company. Following this step, a confirmation by the insurance company may be required. According to an embodiment of the invention, after every step, the available information is stored anew creating a redundancy in the stored information. The further information transmittal is thus made independent of the reliability of the used communication channel. In this way, a concept of 'moving redundancy' - in analogy to the concept of 'moving average' is created. If the operating platform is implemented by a computer server system, this moving redundancy may be accomplished by an according session management.

### BRIEF DESCRIPTION OF THE DRAWINGS

- Fig. 1 shows an overview on a system according to the invention.
- Fig. 2 shows a scheme of an embodiment comprising an operating platform implemented as a server system.
- Fig. 3 represents a scheme of the session management using the operating platform of Fig. 2.
- Fig. 4 shows an embodiment of a method step for transmitting information from a user A to a user B in a secure manner wherein the legibility of information only for the authorized party is assured,
- Fig. 5 represents a functional model of the system and method according to the invention, and
- Fig. 6 shows a scheme of an embodiment of the invention somewhat different from the embodiment shown in Fig. 2.

### DESCRIPTION OF THE PREFERRED EMBODIMENTS

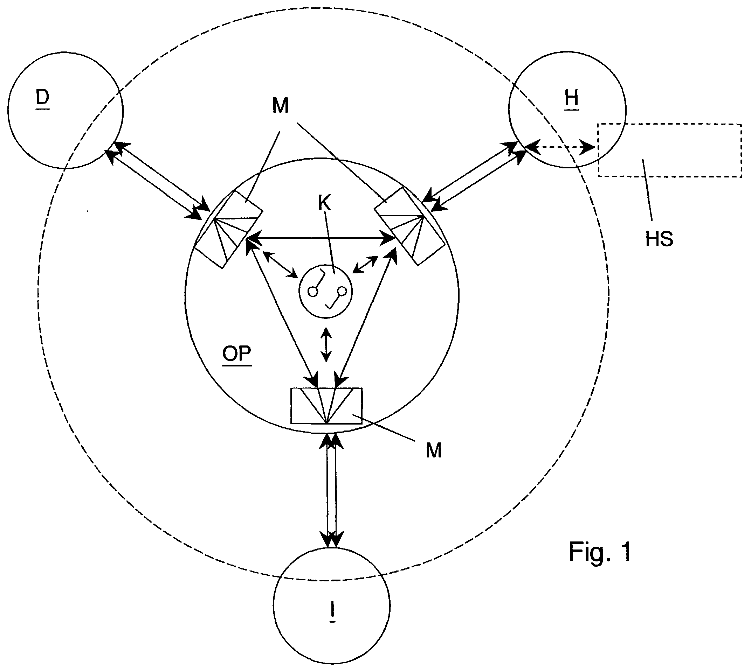

Fig. 1 represents a very schematical overview on the system according to the invention. For reasons of simplicity, only one representant of each, doctors D, Hospitals H and Insurance Companies I is shown. The system comprises an operating platform OP, and communication means between the operating platform and the participants D, H, I. In the drawings, the communication means are symbolized by arrows. According to the embodiment shown, the communication platform comprises information multiplexers M. These multiplexers M comprise means for distributing information between different channels. These different channels may be communication channels between different participants. They also may - optionally - concern different security levels and/or different urgency levels. As an example, a booking request of a patient waiting for a cosmetic operation may be attributed a low urgency level but a moderate or high security level (discretion). In contrast, an offer for an organ transplant will have a very high urgency level.

For instance, the multiplexers contain means for intelligent multiplexing. Multiplexing is done depending on the content of the received information.

The operating platform may also contain de-multiplexers for combining signals from different channels in one channel before they are transmitted to a participant. Depending on the implementation of the multiplexers, the multiplexers themselves may comprise means for forming these de-multiplexers.

The multiplexers M may be implemented by computer software on a computer server system. As an alternative, they also may be implemented by analog electronics. As an example, signals for different channels may be sent, by the participant, modulated on different carrier frequencies. The multiplexer forwards the signal to a channel depending on the carrier frequency. Concerning the actual implementation of this embodiment, it is referred to standard electronic textbooks.

In the following, some aspects of an embodiment of the invention, in which the operating platform is implemented by a computer server system, are discussed.

The system further to the platform also comprises client systems for every participant, i.e. every hospital, doctor, insurance company etc. The entire system in Fig. 1 is symbolized by a dashed circle. For the communication between the participants and the operating platform can, dependent on the chosen (multiplexed) channel, the system can for instance support three interfaces between the operating platform and users:
- Web Interface: This interface may be used for the internet as chosen communication platform. It e.g. comprises a graphic user interface in a web browser such as the internet explorer® or the like.
- A push notification interface. This interface comprises a tool for informing a user also when he or she is not logged in in the system. It may comprise means for automatically sending an e-mail, for automatically sending a fax message, for sending an SMS or for sending a signal to a hospital information system or a hospital disposition system etc..
- An automation interface. This is a integration platform for integrating front office systems such as hospital disposition systems, hospital information systems (denoted by HS in the figure) etc. This integration is e.g. made possible by using protocol being a standard in the medical field such as the HL7 ("health level 7") norm which is currently widely used in hospitals.

A crucial aspect of the invention is data security. In order to ensure a sufficiently secure data handling, as an example the following points may be implemented.
A. Correct authentification: In order to be able to use the system, a user (participant) needs e.g. the three components UserID, Password, and Smartcard. If any one of these components is missing, access to the system via his client system is denied under any circumstances. The smartcard is an electronic equivalent of a key or of a bunch of keys. It can incorporate a plurality of digital keys. Such digital keys exist as pure software tools. In contrast, a smartcard may feature the advantage to exist physically, which makes a responsible handling of the information more easy. The card can be locked up in a safe or be fastened to an ordinary bunch of keys.
B. Secure data transport: Every communication with the operating platform as to be protected against unauthorized access or intercepting by a third party. This is e.g. made possible by the internet standard SSL/TLS. The optimizer platform is authenticated by a so called Certificate Authority (an official institution) to the user. The user thus can be sure to communicate with the operating platform and with nobody else. This having happened, an electronic key is exchanged and every communication between the user and the optimizer platform is encrypted. The key used therefor is only valid for one session. As an example, a 128bit Cypher Suite may be used.
   The first two steps make sure that only authorized persons may gain access to information. It is, however, a special requirement of the system according to the invention, that information is provided selectively. Different members of the already restricted group of potential users should have access to different information only. It has to be certain that only the persons have access to a piece of information which concerns them. As an example, a doctor will provide information for the hospital and for an insurance company specifically.
C. Therefore, according to a further aspect of the embodiment of the invention, to every piece of information a specific destination attribute is given. The destination attribute contains information about the destination of the data to be transmitted as well as possibly also security relevance and urgency information. This destination information is used by the multiplexers M to distribute the signals to the correct channels. Each participant or some participants such as the hospitals may have the opportunity to make configurations determining or influencing the channel depending on the circumstances and on the message content. As an example, a configuration by a hospital may include that certain information is preferably sent to a communication server of a hospital server system. A channel then may be simply represented by this server's IP address. The destination information, however, is also used for implementing a destination specific encryption to be described further below. This concept may e.g. make use of the well known public key infrastructure system. The public key registry according to this concept is denoted by K in the figure.

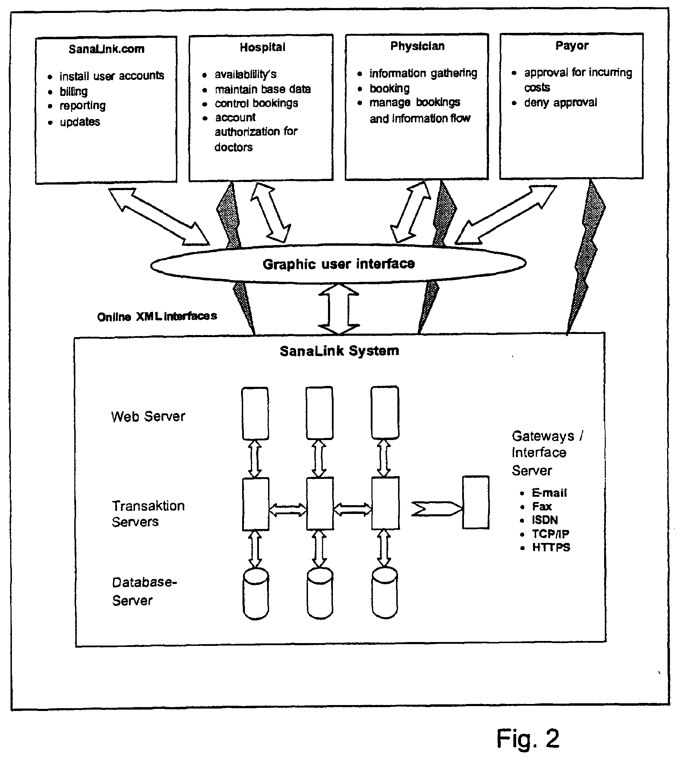

Fig. 2 shows a scheme of an embodiment comprising an operating platform implemented as a server system. The platform denoted by "SanaLink-System" in the figure is connected via a graphic user interface to hospitals, doctors, and insurance companies ("payer"). The platform e.g. three levels: a web server level comprising at least one server with a communication interface, a transaction server level with at least one transaction server and a database server level comprising at least one database server. The transaction servers are responsible for linking the web servers to the databases and for controlling the messages issued and received by the web servers. They may serve a further gateway for different communication interfaces (push notification, etc, see above). In this embodiment, they in this way implement the multiplexing means for distributing data to different channels.

In the figure, also the possibility to serve a internet page ("SanaLink.com") by the platform is indicated.

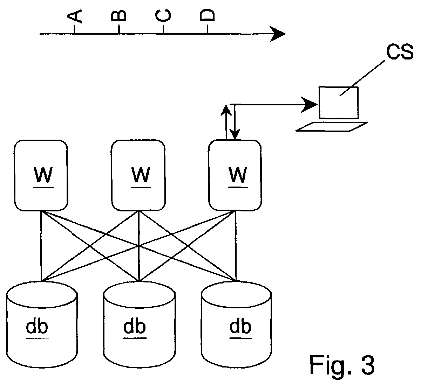

In Fig. 3, an aspect of the server system and of the method is shown in more detail. The shown aspect concerns the concept of moving redundancy implemented by an appropriate session management. In the figure, for reasons of simplicity the transaction servers distributing and managing the data flow between the databases db and the web servers w are left out. As symbolized in the figure, if a participant would like to transmit relevant data, different steps are carried out. As an example, if a doctor would like to reserve a hospital bed and medical attention for a patient, in a first step A he may request and consider information about the hospital occupancy, the offered services and specialties etc. of a plurality of hospitals. In a next step B he e.g. chooses a particular hospital and a possibly also a particular medical service, i.e. a hospital service such as a consultation, a check or a treatment. Then he fills in a form with the relevant data (step C) and transmits the data as a request signal to the operating platform (step D). According to this embodiment of the invention, for every step a new connection between a web server w and the client system CS is established. After every step, all the already available data (e.g. the identity of the doctor, the criteria, according to which he selects a hospital etc.) are transmitted to the databases db and stored. This redundant data storage effects that the transmission security is not crucially affected by the security and reliability of an actual data transmission line. If an action fails, the relevant data are stored in the databases. A new connection may be opened and the transaction may be continued at the point where the failure happened. Due to the construction of the server system, it is not a requirement that the connection is established from the same web server.

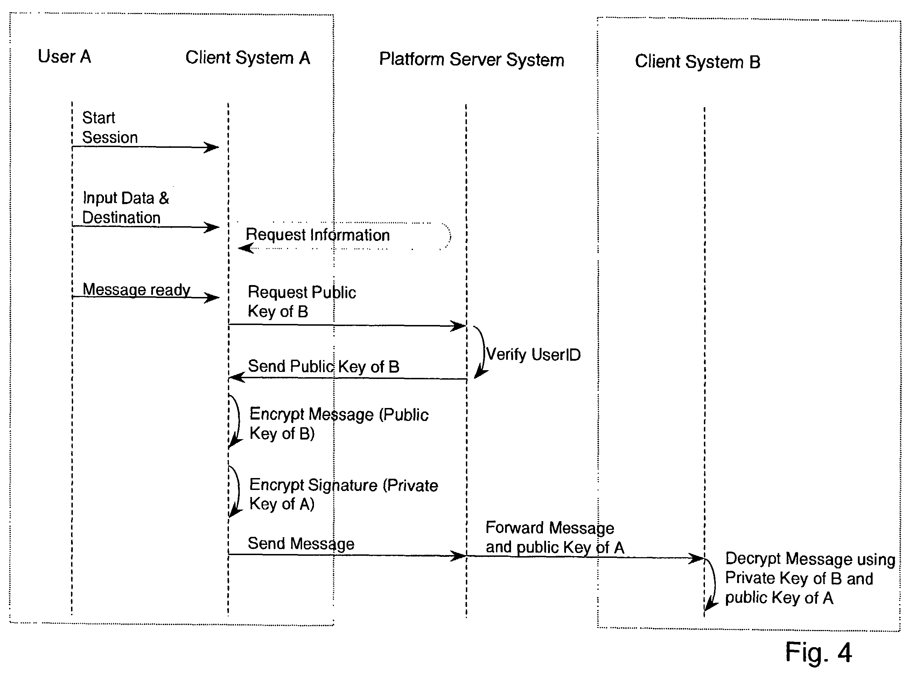

Next, an example of an implementation of specific encryption is described with reference to Fig. 4. Fig. 4 shows a sequence diagram of a simplified communication process between a user A (e.g. a doctor) and a user B (e.g. a hospital). The communication between the client system A and the operating platform server system as well as between the operating platform server system and the client system B are e.g. 128 bit encrypted. The information exchanged between the operating platform and the server system ("request information") in the beginning after the user has started the session are e.g. of a general nature. It may comprise data about the hospital occupancy, the doctors on duty and their specialties, other services etc. They are therefore of a relatively low security relevance. Thus it is not necessary that they are specifically encrypted, the mentioned 128 bit encryption is sufficient. However, data compiled by user A afterwards ("Message ready") comprises security relevant information including patient data. It has to be encrypted destination specifically. To this end, the client system A requests B's specific key data from the platform, e.g. B's public PGP key. The fact that the transmitted data are B encrypted makes sure that not even a person having access to the operating platform can read the transmitted data.

By encrypting his digital signature with his private key, A gives the recipient the opportunity to verify that the information really stems from A by decrypting the signature with the public key of A.

If information is not only to be sent to one destination but to a plurality of destinations (e.g. including an insurance company) the user program A may request a plurality of public keys from the platform and individually encrypt the different pieces of information according to their destination. Depending on the destination attribute, the information is forwarded to different participants

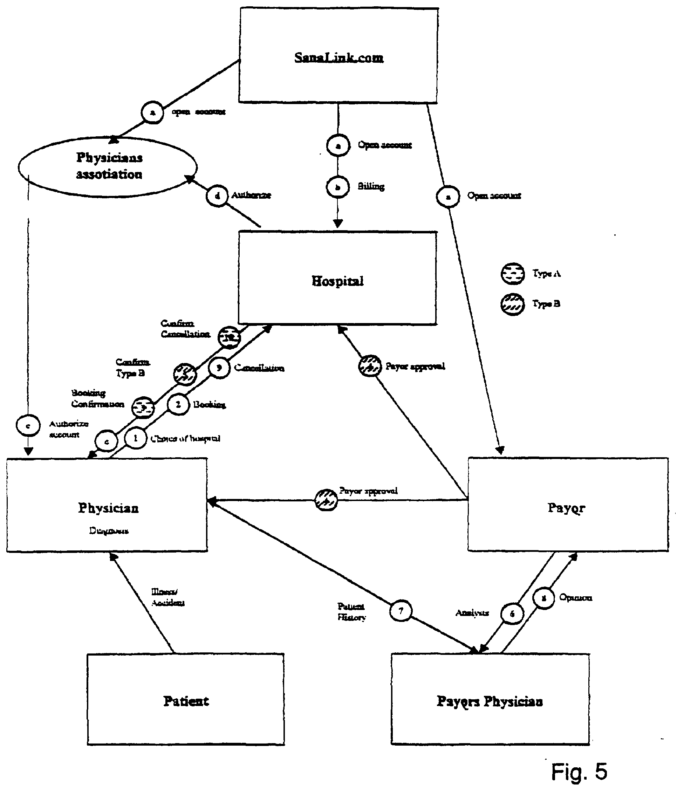

Fig. 5 shows an information flow scheme of a special embodiment with more user categories (Hospitals, Doctors, Insurance Companies (Payers) as well as a platform related webpage (Sanalink.com), doctors associations etc.). The plurality of different information types and information channels requires a multiplexing and selecting of information including a plurality of keys or the like etc. For reasons of simplicity, the operating platform is not shown in the figure.

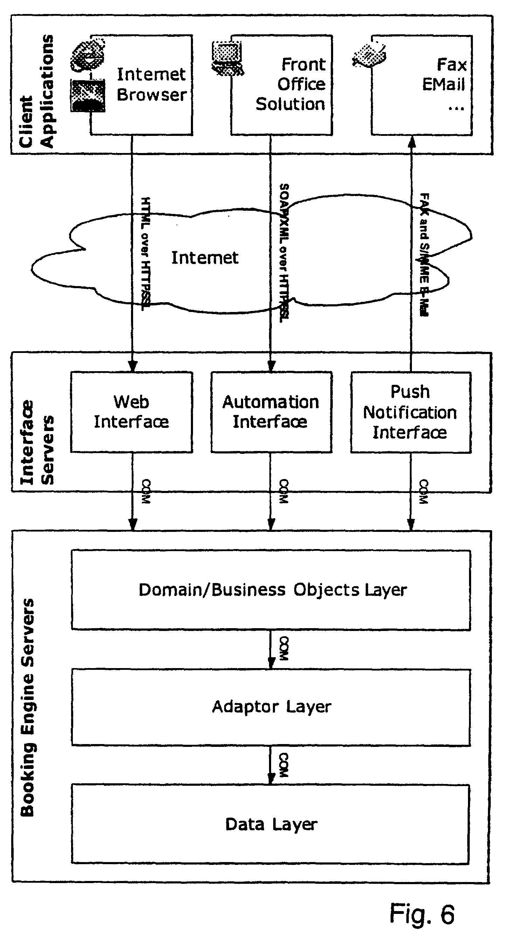

Fig. 6, finally, shows an embodiment somewhat different to the one presented in Fig. 2. In this figure, the three different interfaces referred to above are represented. The operating platform comprises interface servers and 'booking engine' servers. The interface server level corresponds in function to the web server level of Figure 2. The data base means for storing information such as a compilation of public keys, hospital and other participant related information etc. and also the session information in the way described with reference to Fig. 3 are formed by a data layer. A domain/business objects layer and an adapter layer in between serve for processing and administering data, commands etc.

It should be appreciated that the above described embodiments are by no means the only way to realize the invention but can be altered in many ways. As a first example, also conventional, symmetric digital encryption can be used with an encryption key specific to the destination and dependent on the actual time. Also such a key can be administered by the operating platform. As a further example, also a destination specific encryption can be realized in an analog technology. In this context it is referred to encryption techniques using numeric or other keys known for decades already.

Various other embodiments may be envisaged without departing from the spirit and scope of the invention.

## Claims

1. A method of exchanging information between a plurality of doctors, a plurality participants, at least some of them being hospitals and other ones of them being doctors, comprising the steps of
maintaining an operating platform and communication means linking said operating platform to the doctors and hospitals,
transmitting message data containing a destination information from a participant to said operating platform,
distributing, using multiplexing means and data contained in the destination information, said message data to at least one of a plurality of channels, the channels being at least distinct in the information destination and in the security and/or urgency levels, and
transmitting said message data to at least one participant via said channels using said communication means.

2. A method as claimed in claim 1 wherein, before message data containing a destination information is transmitted from a participant to said operating platform, destination information is sent to the platform and an encryption key is requested, wherein an encryption key associated with a message recipient specified in the destination information is read from an encryption key database of the platform and transmitted to the participant, and wherein the message to be sent is encrypted by the participant using this one encryption key.

3. A method as claimed in claim 1 or 2 wherein the message data to be transmitted is compiled by the participant in several steps, wherein for at least some of the steps, a communication link to the operating platform is established, and after the step the entire data transmitted so far is stored in the operating platform's storage means and the communication link is closed.

4. A method of exchanging information between one of a plurality of doctors and one of a plurality of hospitals comprising the steps of
maintaining a set of encryption keys individually relating to each of the plurality of hospitals by the operating platform
opening a communication link between a client system of said one doctor and an operating platform
sending an encryption key request signal specifying said one hospital from the said client system to said operating platform
sending an encryption key specific to said one hospital from the operating platform to the doctor
encrypting the data to be transmitted using said specific encryption key
sending the thus encrypted data from the doctor's client system to the operating platform and forwarding the data from the operating platform to a client system of the hospital.

5. A method as claimed in claim 4, wherein on the operating platform, a data set containing hospital related information including information about hospital occupancy is maintained and wherein said one hospital is selected by the doctor according to information from said data set and transmitted to the doctor's client system.

6. A server system for establishing an operating platform system for exchanging information between a plurality of doctors and a plurality of hospitals comprising at least one computer server with data storage means, data processing means and communication means for establishing a communication link to each of said doctors and hospitals wherein the data storage means comprise computer program code implementing in the server system the following means:
- means maintaining and regularly actualizing a data set containing hospital related information including information about hospital occupancy and/or available hospital services
- means for transmitting data in including data about hospital occupancy and/or available hospital services to a client system of a doctor
- multiplexing means for message data to at least one of a plurality of channels, the channels being at least distinct in the information destination and in the security and/or urgency levels, and
- means for transmitting said message data to at least one participant via said channels using said communication means.

7. A method of exchanging information between one of a plurality of doctors and one of a plurality of hospitals comprising the steps of maintaining, on an operating platform, a data set containing hospital related information including information about at least one of hospital occupancy and of services available from the hospital,
displaying, on a client system of said one doctor, information relating to at least some of the plurality of hospitals including information from said data set in response to an action being performed by the doctor, sending a request signal to said operating platform
generating a reservation order by said operating platform
sending said reservation order to one of said plurality of hospitals, the hospital being specified by information contained in the request signal, and
depending on the successful transmittal of said reservation order, modifying said data set.

8. A method as claimed in claim 7, wherein further to the reservation order a request for a payment precertification is generated by the operating platform and
wherein this request is sent to an insurance company specified upon information contained in said request signal.

9. A method as claimed in claim 8 wherein the operating platform can be configured in a manner that said reservation signal is only transmitted if the request for a payment precertification is positively answered to by the insurance company.

10. A method as claimed in any one of the previous claims wherein said operating platform is implemented by a server system.

11. A method according to any one of the previous claims wherein further to the reservation request also medical data such as diagnosis, x-ray or CT images etc. are transmitted.

12. A system for exchanging information between a plurality of doctors and a plurality of hospitals comprising
an operating platform comprising data storage means and data processing means for maintaining a data set with hospital related information including
information about hospital occupancy and communication means for establishing a communication link to each of said doctors and hospitals
for each doctor, a client system comprising communication means for establishing a communication link to said operating platform, a display component and means for generating a request signal to said operating platform,
and, for each hospital, a client system comprising communication means for establishing a communication link to said operating platform, and means for receiving a reservation signal from said'operating platform.

13. A server system for establishing an operating platform system for exchanging information between a plurality of doctors and a plurality of hospitals comprising data storage means, data processing means and communication means for establishing a communication link to each of said doctors and hospitals wherein the data storage means comprise computer program code for implementing means for maintaining a data set with hospital related information including information about hospital occupancy, means for transmitting hospital related information to a doctor, means for receiving a request signal from a doctor, means for generating, depending on the request signal, a reservation signal and sending the same to a hospital and means for modifying said data set with hospital related information depending on the successful transmission of said reservation signal.
